# EUROPÄISCHE PATENTSCHRIFT

(11) **EP 0 734 524 B1**
(45) Veröffentlichungstag und Bekanntmachung des Hinweises auf die Patenterteilung: **21.05.1997**
(21) Anmeldenummer: 95936492.8
(22) Anmeldetag: 13.10.1995
(51) Int. Cl.: G01N 33/49

(54) **VORRICHTUNG ZUM MESSEN DER KOAGULATIONSEIGENSCHAFTEN VON TEST-FLÜSSIGKEITEN**
DEVICE FOR MEASURING THE COAGULATION PROPERTIES OF TEST LIQUIDS
DISPOSITIF DE MESURE DES PROPRIETES DE COAGULATION DE LIQUIDES UTILISES POUR DES TESTS

(30) Priorität: 19.10.1994 DE 4437475; 04.04.1995 DE 29505764 U
(43) Veröffentlichungstag der Anmeldung: 02.10.1996
(73) Patentinhaber: Calatzis, Alexander, 80634 München (DE); Calatzis, Andreas, 80634 München (DE); Fritzsche, Pablo, 80799 München (DE)
(72) Erfinder: Calatzis, Alexander, 80634 München (DE); Calatzis, Andreas, 80634 München (DE); Fritzsche, Pablo, 80799 München (DE)
(74) Vertreter: Prüfer, Lutz H., Dipl.-Phys.
(86) Internationale Anmeldenummer: EP9504041
(87) Internationale Veröffentlichungsnummer: WO9612954

(56) Entgegenhaltungen:
- EP-A- 0 404 456
- DE-A- 3 738 901
- FR-A- 2 389 137
- US-A- 3 053 078
- IEEE TRANSACTIONS ON BIOMEDICAL ENGINEERING, Bd. BME-33, Nr. 9, September 1986 NEW YORK, US, Seiten 887-890, SHOUPU CHEN 'A MICROPROCESSOR-BASED TWO-CHANNEL THROMBOELASTOGRAPH'

## Beschreibung

Die Erfindung betrifft eine Vorrichtung zum Messen der Koagulationseigenschaften von Test-Flüssigkeiten.

Eine Vorrichtung zum Messen der Koagulationseigenschaften von Test-Flüssigkeiten ist aus der DE-845 720 und DE-U 76 16 452 bekannt. Bei dieser Vorrichtung ist ein Stab an einem Torsionsdraht frei hängend aufgehängt und an seiner Unterseite mit einem Stempel versehen, der zum Zwecke der Messung der Koagulationseigenschaften einer Test-Flüssigkeit in eine die Test-Flüssigkeit aufnehmende Küvette eingetaucht wird. Üblicherweise befinden sich in der Küvette 0,36 ml einer Test-Flüssigkeit, beispielsweise Blut. Während der Messung wird die Küvette in einer Sinusbewegung um 4,75° nach rechts und links gedreht, wobei eine Periode 10 sec. beträgt. Die Küvette hat üblicherweise einen Durchmesser von 8 mm, der Stempel einen Durchmesser von 6 mm. Die Messung basiert darauf, daß die Drehung der Küvette nicht auf den Stempel übertragen wird, solange keine Verbindung zwischen Stempel und Küvette vorhanden ist. Wenn sich eine Verbindung, zum Beispiel mittels Netzwerken von Fibrinfäden und Blutplättchen, bildet, folgt der Stempel mehr und mehr der Rotationsbewegung der Küvette. Das sich bildende Netzwerk versucht einem Auslenkwinkelunterschied zwischen Stempel und Küvette entgegenzuwirken. Dadurch wird ein Drehmoment von der Küvettendrehung auf den Stempel übertragen, und zwar in um so höherem Maße, je stärker das Netzwerk zwischen Stempel und Küvette ausgeprägt ist. Dagegen wirkt der Torsionsdraht einer Auslenkung des Stempels aus seiner Nullposition entgegen. Es stellt sich mithin ein Momentengleichgewicht zwischen dem Rückstellmoment des Torsionsdrahtes und dem auf den Stempel wirkenden Drehmoment ein, wobei Trägheitskräfte sowie viskose Kräfte zu vernachlässigen sind, da die Bewegungen sehr langsam ablaufen. Die Amplitude der Stempelbewegung stellt also ein maß für die Stärke des sich bildenden Netzwerkes bzw. Gerinnsels dar. Die Amplitude wird genau gemessen und als Funktion der Zeit aufgetragen. Bei der nach dem Stand der Technik bekannten Vorrichtung hängt der Stempel wie ausgeführt über den zugehörigen Stab frei am Torsionsdraht, welcher das oben benannte Rückstellmoment ausübt. Damit ergibt sich eine hohe Empfindlichkeit gegen Vibration und Translationsbewegungen am Meßgerät, die die Messungen verfälscht und der man dadurch entgegenzuwirken versucht, daß der Stab seitlich abstrebende Flügel aufweist, die in eine mit Öl gefüllte, gekammerte Wanne abgesenkt werden. Wie in der DE-U-76 16 452 ausgeführt, ist die Empfindlichkeit gegenüber Störeinflüssen trotz der Einrichtung der genannten Ölwannen und Flügel so hoch, daß eine optische Abtastung des Drehwinkels des Stempels mit den bekannten Vorrichtungen gestört ist. In der genannten Anmeldung wird ein aufwendiger induktiver Differential-Transformator-Winkelgeber beschrieben, der Signalstörungen durch nicht rotationsbedingte Bewegungen des Stempels verringern soll. Die bekannten Vorrichtungen bestehen aus einer Vielzahl von individuell gefertigten Teilen, sind damit kostenintensiv, weiterhin empfindlich gegen Stöße, weil das Meßsystem jeweils frei hängt. Außerdem sind die bekannten Vorrichtungen umständlich zu bedienen, da die Öl enthaltenden Kammern gefüllt werden müssen und die Systeme exakt in der Waage zu stehen haben. Der Transport einer derartigen Vorrichtung ist äußerst umständlich, insbesondere müssen die Öl enthaltenden Kammern vorher entleert werden und vor erneutem Betrieb wieder gefüllt werden.

Aus der GB 1353 481 (Fig. 2) sind weitere Vorrichtungen zur Messung der Koagulationseigenschaften von Blutproben bekannt.

Der Erfindung liegt die Aufgabe zugrunde, eine Vorrichtung der eingangs genannten Art dahingehend zu verbessern, daß sie stabiler und unempfindlicher ist, sowie einfacher herzustellen.

Diese Aufgabe wird erfindungsgemäß durch den Gegenstand des Patentanspruches 1 gelöst.

Weitere Ausgestaltungen der Erfindung ergeben sich aus den Unteransprüchen.

Gemäß der Erfindung ist ein mechanisches Führungssystem vorgesehen, welches die Freiheitsgrade des Stempels auf die Rotation um die Hochachse bzw. Vertikale begrenzt.

Die erfindungsgemäße Vorrichtung gewährleistet eine nahezu reibungskraftfreie Funktion des mechanischen Führungssystems, vorzugsweise in Form eines Kugellagers. Dadurch ist bei einer derartigen Vorrichtung, auch zu dem Zeitpunkt eine exakte und fehlerfreie Bewegung des Stempels möglich, an welchem das Blutgerinnsel nur eine äußerst geringe Kraft auf den Stempel überträgt. Dies ist überaus bedeutsam, da ein wichtiger Parameter derartige Vorrichtungen, insbesondere bei der Anwendung in der Thrombelastographie, die Reaktionszeit ist, das heißt die Zeit vom Beginn der Messung bis zu dem Zeitpunkt, an welchem der Stempel ca. 1/100 der Küvettenbewegung von 4,75°, entsprechend weniger als 3 Bogenminuten, vollführt. Gemäß der Erfindung ist eine exakte Messung der Bewegung des Stempels bereits bei sehr kleinen Winkeln bzw. sehr kleinen Kräften gesichert. Der Aufbau der Vorrichtung selbst im Bezug auf die Führung des Stempels ist einfach, mit wenig Aufwand verbunden und störunanfällig.

Weitere Merkmale und Zweckmäßigkeiten ergeben sich aus der Beschreibung von Ausführungsbeispielen anhand der Figuren.

Von den Figuren zeigen:
- Fig. 1: eine perspektivische Darstellung von Welle und mechanischer Führungseinrichtung eines ersten Ausführungsbeispieles, von vorne gesehen,
- Fig. 2: eine Fig. 1 entsprechende Darstellung als Rückansicht,
- Fig. 3: eine Fig. 1 entsprechende Darstellung, von unten gesehen,
- Fig. 4: eine perspektivische Ansicht eines Teiles einer erfindungsgemäßen Vorrichtung des ersten Ausführungsbeispieles,
- Fig. 5: eine perspektivische Teildarstellung einer Meßstation des ersten Ausführungsbeispieles,
- Fig. 6: eine schematische perspektivische Darstellung eines zweiten Ausführungsbeispieles,
- Fig. 7: eine Schnittdarstellung entlang der Linie A-B in Fig. 6
- Fig. 8: eine perspektivische Darstellung einer Meßstation des zweiten Ausführungsbeispieles.

### Erstes Ausführungsbeispiel:

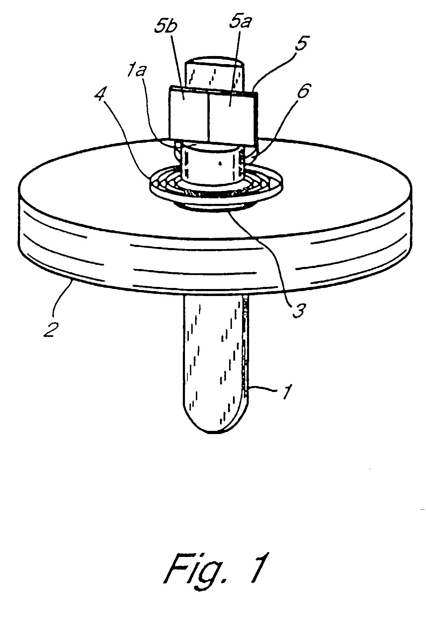

Der erfindungsgemäßen Vorrichtung zugrunde liegt eine Abtastmechanik mit mechanischer Führungseinrichtung und geführter Welle gemäß den Fig. 1-3.

Die Fig. 1-3 zeigen die bei der erfindungsgemäßen Vorrichtung verwendete mechanische Führungseinrichtung 3 und die von ihr gehaltene Welle 1. Am unteren Ende der Welle 1 kann ein nicht dargestellter Kunststoffstempel angehängt werden, der bei der Messung mit dem Blut reagiert. Gemäß den Fig. 1 bis 3 sitzt die Welle 1 drehfähig in der mechanischen Führungseinrichtung 3. Diese Führungseinrichtung ist vorzugsweise ein Wälzlager. Dieses soll möglichst reibungsfrei arbeiten. Ein Kugellager, z.B. Rillenkugellager mit geringem Durchmesser, beispielsweise 3 mm Durchmesser, eignet sich hier besonders zum Einsatz. Mit der Welle 1 verbunden sind der am unteren Teil der Welle angehängte Stempel (nicht dargestellt), eine Einrichtung zur Winkelabtastung, beispielsweise in Form eines Spiegels 5, der bei einer bevorzugten Ausführungsform entsprechend den Fig. 1 bis 3 an dem oberen Ende der Welle 1 angeordnet ist, eine Einrichtung zur Erzeugung eines Rückstellmoments, vorzugsweise in Form einer Spiralfeder 4 und der rotierende Teil des Kugellagers 3. Diese Teile drehen sich während der Messung mit der Welle und besitzen zusammen mit der Welle, um eine möglichst reibungsfreie Drehung zuzulassen, eine äußerst geringe Masse, vorzugsweise weniger als ein Gramm.

Das Kugellager ist in eine Scheibe oder Platte 2 eingesetzt.

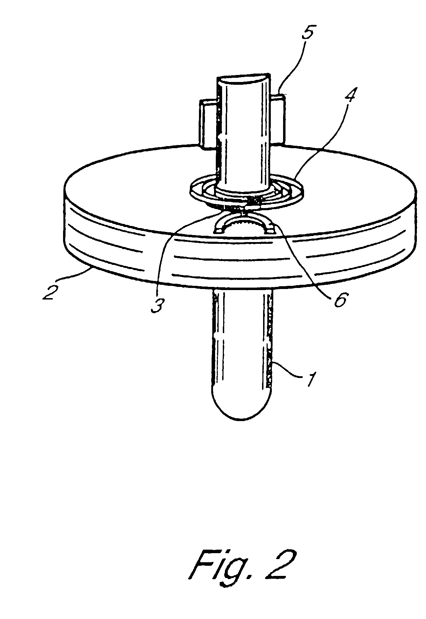

Soweit eine Spiralfeder 4 zur Erzeugung eines Rückstellmomentes in bezug auf die Welle 1 vorhanden ist, ist ein Ende der Spiralfeder fest mit der oberen Hälfte der Welle 1 verbunden, wie Fig. 2 zeigt, während das andere Ende der Spiralfeder 4 gegenüber der Platte oder Scheibe 2 fixiert ist.

Die Spiralfeder 4 liegt vorzugsweise in einer Ebene oberhalb und parallel zum Kugellager 3. An der Scheibe oder Platte 2 sind Befestigungsmittel 6 vorgesehen, in welche das eine Ende der Spiralfeder 4 fest eingesetzt wird, z.B. in Form eines Haltenockens oder -zapfens. An der Welle 1 ist zum Beispiel ein Schlitz zur Aufnahme des anderen Endes der Spiralfeder 4 ausgebildet.

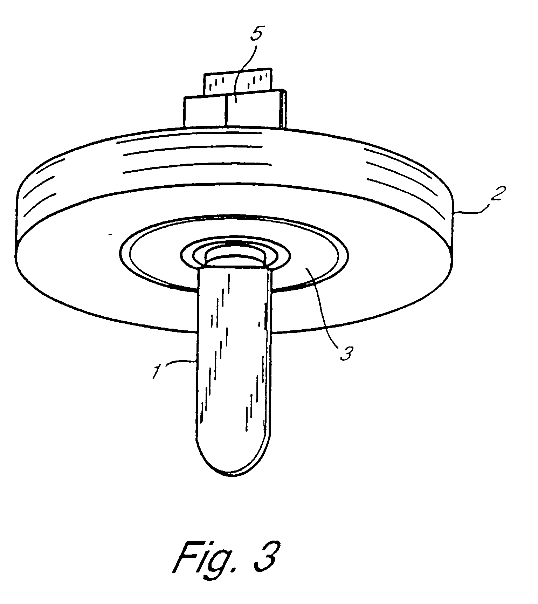

Aus Fig. 3 wird der Einsatz des Kugellagers deutlich, während Fig. 1 und 2 die Anwendung einer Spiralfeder 4 veranschaulichen, welche gegenüber der Welle 1 ein Rückstellmoment ausübt.

Der Spiegel 5 ist derart in die Welle 1 eingesetzt, daß er durch einen Abtaststrahl über die aktuelle Drehung der Welle 1 eine Information liefert. Am oberen Ende der Welle 1 ist gemäß Fig. 1 hierfür ein Teil der Welle 1 abgefräst. Mit der auf diese Weise gebildeten Stufe 1a als untere Auflage nimmt die Welle 1 den Spiegel 5 gemäß der Fig. 1 auf.

Die Welle ist relativ kurz im Vergleich zu den beim Stand der Technik verwendeten Stäben, besteht aus leichtem Material wie beispielsweise Aluminium oder Keramik oder dergleichen. Die Welle 1 wird vorzugsweise etwa in der Mitte ihrer Länge von dem Kugellager 3 geführt.

Zur Gewährleistung einer möglichst geringen Masse des vorstehend erläuterten rotierenden Systems, wird der Drehwinkel der Welle über den Spiegel 5, vorzugsweise einen kleinen Planspiegel, abgetastet. Die Abtastung erfolgt vorzugsweise mittels eines kollimierten, idealerweise strichförmigen Lichtstrahls unter Verwendung eines Plan- oder Konkavspiegels 5 und einem CCD-Zeilensensor.

Gemäß einer weiteren Ausgestaltung der Erfindung, wird eine nichtkollimierte Lichtquelle, z.B. in Form einer Diode, als Strahlenerzeuger eingesetzt und als Spiegel 5 ein Planspiegel, der zum Teil mit einer undurchsichtigen Folie 5a beklebt ist. Der Rand zwischen spiegelnder Fläche 5b und nicht spiegelnder Fläche 5a wird an dem CCD-Zeilensensor äußerst genau und rauschfrei abgebildet und der entsprechende Winkel nach Abtastung des CCD-Zeilensensors vorzugsweise unter Einsatz einer entsprechenden Software berechnet.

Die in den Fig. 1 bis 3 gezeigte Einheit weist eine äußerst einfache Konstruktion auf, erfordert wenig Teile und es können Standardkomponenten, das heißt Kugellager, und so einfache individuelle Teile, wie Spiralfeder, rechteckiger Spiegel, usw. verwendet werden, was den kostenmäßigen Aufwand erheblich reduziert und die Zuverlässigkeit des gesamten Systems maßgeblich verbessert.

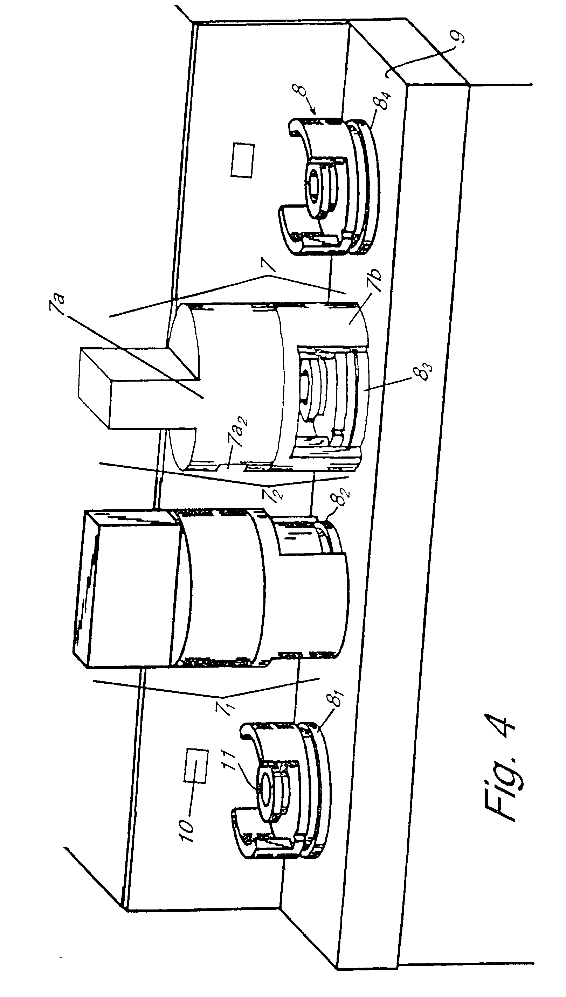

Figur 4 zeigt eine perspektivische Teilansicht einer bevorzugten Ausgestaltung der erfindungsgemäßen Vorrichtung.

Eine Meßeinheit der erfindungsgemäßen Vorrichtung besteht aus einer als Abtastzylinder bezeichneten abnehmbaren Einheit 7 und einem fest ander erfindungsgemäßen Vorrichtung fixierten Gegenstück 8.

Die erfindungsgemäße Vorrichtung besitzt vorzugsweise vier Meßeinheiten, von denen in Fig. 4 zwei komplett dargestellt sind und mit 7₁, 8₂ bzw. 7₂, 8₃ bezeichnet sind. Die Abtastzylinder 7 enthalten die in den Fig. 1 bis 3 dargestellte Meßmechanik und können in noch zu beschreibender Weise auf die Gegenstücke 8 aufgesetzt werden und gegenüber den Gegenstücken 8 in eine Beschickungsposition und eine Testposition verdreht werden. Gemäß einer bevorzugten Ausführungsform stellen die Gegenstücke 8 mit den Abtastzylindern 7 eine Verschlußeinheit dar, die einen Bajonettverschluß ergibt.

Die Basis stellt einen mittels einer Steuerung thermostatisch geregelt auf 36,5° beheizten Block dar, der vorzugsweise aus Aluminium besteht.

Jedes Gegenstück 8 nimmt in seiner Mitte die Küvettenaufnahme 11 auf. In die Küvettenaufnahme 11 wird die Küvette eingelegt, in welche die zu untersuchende Flüssigkeit, vorzugsweise Blut, eingebracht wird. Die Küvettenaufnahmen 11, und mit ihnen die Küvetten, werden auf konventionelle Weise in einer Sinusbewegung um 4,75° nach rechts und links gedreht. Die Küvettenaufnahmen stehen in Kontakt mit der Basis 9 und werden mittels dieser passiv beheizt.

In die Küvettenhalterungen werden vorzugsweise im offenen Zustand, gemäß Fig. 4 (Gegenstück 8₁ und 8₄), die Küvetten und Testflüssigkeiten eingebracht.

Um den in den Fig. 1 bis 4 nicht dargestellten Stempel austauschen zu können, wird der mit 7 bezeichnete Abtastzylinder abgenommen. Zur Einleitung der Messung wird der Abtastzylinder 7 auf das Gegenstück 8 aufgesteckt, wobei der Stempel in die in der Küvette befindliche Testflüssigkeit eintaucht.

In der Perspektivansicht nach Fig. 4 ist der Abtastzylinder 7₂ in der Beschickungsposition dargestellt, d.h. der Abtastzylinder 7₂ ist gegenüber dem Gegenstück 8₃ so verdreht, daß eine Sichtkontrolle der Probe möglich ist, sowie das Abdecken der Probe mit einem Tropfen Öl, um ein Austrocknen der Probe während der Messung zu vermeiden.

Der Abtastzylinder 7 läßt sich aus der Beschickungsposition gegenüber dem Gegenstück 8 dahingehend verdrehen, daß das an dem Abtastzylinder ausgebildete Fenster 7a₂, direkt vor einem mit 10 bezeichneten Fenster in der Gehäuserückwand steht. Dies ist die Testposition gemäß Gegenstück 8₂ und Abtastzylinder 7₁. In dieser Position ist der Durchgang eines Lichtstrahles oder dergleichen von einem Strahlenerzeuger hinter dem Fenster 10 zu dem Spiegel an der Welle im Abtastzylinder 8 und zurück in das Gehäuseinnere möglich. Im Gehäuseinneren ist der CCD-Zeilensensor für eine Abtastung des vom Spiegel zurückgeworfenen Lichtstrahles untergebracht.

Ein wesentlicher Vorteil der erfindungsgemäßen Vorrichtung ergibt sich dadurch, daß sämtliche Abtastzylinder 7, sowie Küvettenhalterungen 11 auf einer gemeinsamen beheizten Basis 9 angeordnet sind und damit passiv beheizt werden.

Ein wesentlicher Vorteil der erfindungsgemäßen Vorrichtung ergibt sich dadurch, daß durch die Fixierung der in den Fig. 1 bis 3 gezeigten Einheiten in dem Abtastzylinder 7, die Entfernung der Welle 1 mit Stempel aus der Küvette auf einfache Weise manuell bewerkstelligt werden kann und eine aufwendige Mechanik zum Heben/Senken der Meßmechanik entfällt.

Das vorstehend erläuterte Abtastsystem mit Spiegel 5 und entsprechenden Lichtstrahlen kann auch durch andere Abtastsystem ersetzt werden.

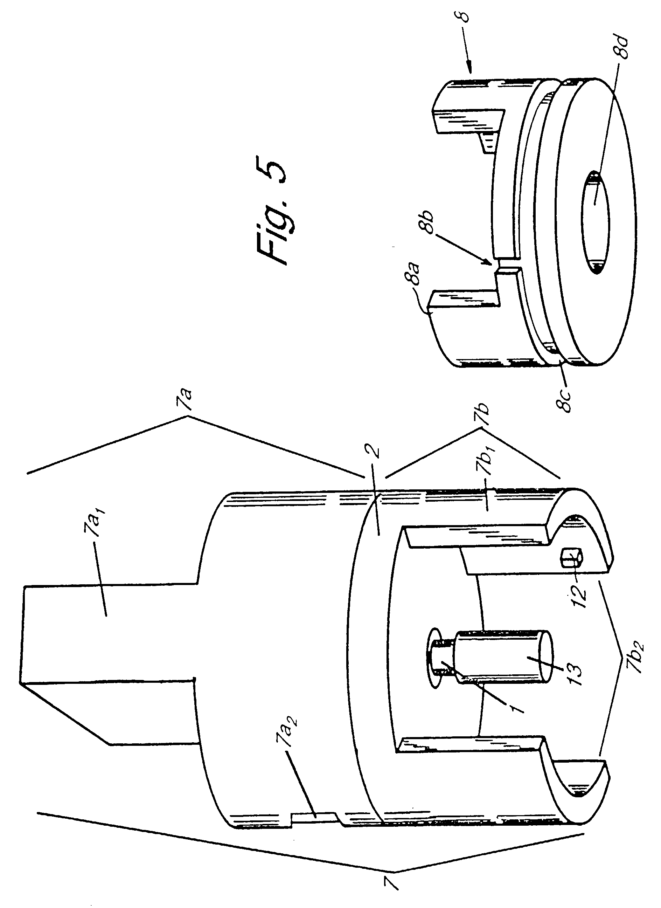

Fig. 5 zeigt Einzelheiten des Abtastzylinders 7 und des Gegenstückes 8.

Jede als Abtastzylinder bezeichnete Einheit 7 besteht aus zylindrischen Teilen 7a und 7b. Der obere Zylinder 7a besitzt an seiner oberen Seite einen Griff 7a₁ und ist vorzugsweise aus Kunststoff hergestellt. Der Zylinder 7a ist innen hohl ausgebildet und besitzt seitlich ein Fenster 7a₂, das in der Testposition gegenüber dem Fenster 10 (Fig. 4) ausgerichtet ist.

Der untere Zylinder 7b besitzt zwei seitliche teilkreisförmige Wände 7b₁ und zwei entsprechende Öffnungen 7b₂. Oberhalb der teilkreisförmigen Wände 7b₁ besitzt der Zylinder 7b die Scheibe 2, in die gemäß den Fig. 1 bis 3 das in Fig. 5 nicht dargestellte Kugellager mit geführter Welle 1 eingesetzt ist. Oberhalb der Scheibe 2 ragt der in den Fig. 1 bis 3 dargestellte obere Teil der Welle 1 mit Spiralfeder 4 und Spiegel 5 in den oberen Zylinder 7a hinein. Zwischen den teilkreisförmigen Wänden 7b₁ ragt die Welle 1 mit dem an ihrem unteren Ende angehängten Stempel 13 hervor. Der Stempel ist aus Kunststoff oder dergleichen hergestellt und ist bei Betrieb in die Küvette eingeführt. Der untere Zylinder 7b ist vorzugsweise aus Aluminium hergestellt, wodurch er gemäß der Fig. 4 die Wärme von der thermostatisierten Basis 9 aufnimmt und passiv beheizt wird.

Das in Fig. 5 dargestellt Gegenstück 8 ist ebenfalls zylindrisch und weist einen Außendurchmesser auf, der dem Innendurchmesser des Abtastzylinders 7 entspricht, so daß der Abtastzylinder 7 auf das Gegenstück 8 aufgesteckt werden kann. Dabei wird der an den teilkreisförmigen Wänden 7b₁ des Abtastzylinders 7 befestigte Stift 12 durch den Schlitz 8b in die Ringnut 8c des Gegenstückes 8 eingeführt. Der Abtastzylinder 7 kann in der aufgesteckten Position gegenüber dem Gegenstück 8 verdreht werden. Die Position, in der die am Gegenstück 8 ausgebildeten Teilkreisförmigen Wände 8a hinter den am Abtastzylinder 7 ausgebildeten teilkreisförmigen Wände 7b₁ stehen und somit von diesen verdeckt werden, ist die Beschickungsposition. In dieser Position besteht freie Sicht auf die Probe. Durch eine Drehung um 90° wird der Abtastzylinder in die Testposition gebracht, wobei der Verschluß zwischen Abtastzylinder und Gegenstück in einer exakten Position einrastet. In dieser Position stehen die teilkreisförmigen Wände 7b₁ und 8a auf Lücke, d.h. jeweils nebeneinander und schließen das Luftvolumen zwischen Abtastzylinder und Gegenstück weitgehend ab, so daß ein Abkühlen der Testflüssigkeit während der Messung durch Konvektion oder Zugluft verhindert wird. Eine Abkühlung der Testflüssigkeit während der Messung würde die beispielsweise während der Blutgerinnung ablaufenden Prozesse verlangsamen und das Ergebnis der Messung verfälschen. Das Gegenstück 8 ist vorzugsweise aus Kunststoff hergestellt. In seiner Mitte besitzt das Gegenstück 8 eine kreisrunde Aussparung 8d, die die in Fig. 4 dargestellte Küvettenfassung 11 aufnimmt.

### Zweites Ausführungsbeispiel:

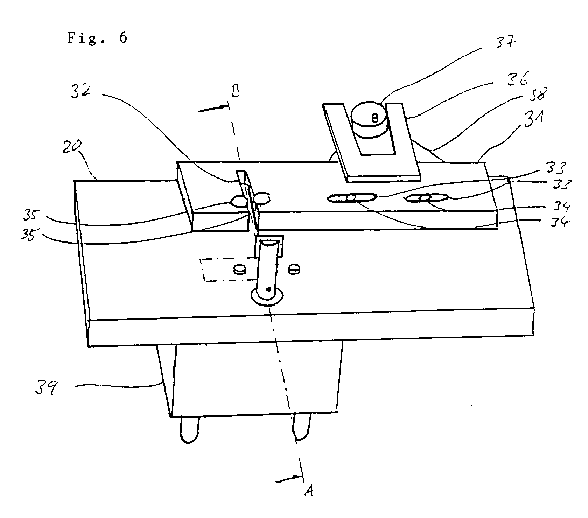

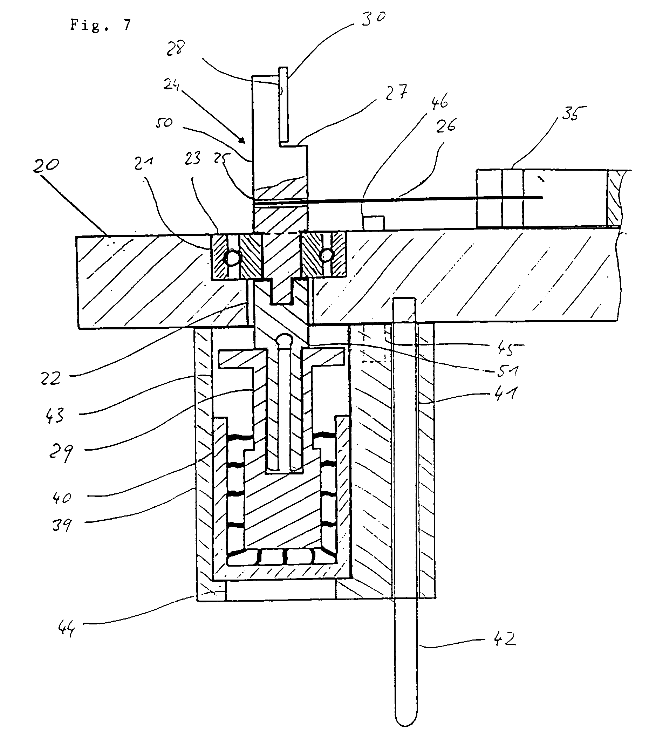

Es ist eine Platte 20 vorgesehen. Wie in Fig. 6 und 7 gezeigt, weist die Platte 20 senkrecht zur Plattenebene eine erste Bohrung 21 mit einem ersten Durchmesser auf. An die erste Bohrung 21 schließt sich eine zweite Bohrung 22 mit einem zweiten Durchmesser, der kleiner als der erste Durchmesser ist, koaxial an. Die Länge der Bohrungen 21, 22 entspricht der Ausdehnung der Platte 20 senkrecht zur Plattenebene. In der ersten Bohrung 21 ist koaxial ein Kugellager 23 angeordnet, dessen Außendurchmesser etwa dem ersten Durchmesser entspricht, und dessen Innendurchmesser kleiner als der zweite Durchmesser ist.

Mit einem Innenring des Kugellagers 23 ist eine im wesentlichen zylinderförmige Welle 24 koaxial verbunden.
Die Welle 24 weist einen oberen ersten Teil 50 und einen mit dem ersten Teil verschraubten unteren zweiten Teil 51 auf.
Das erste Teil 50 weist einen ersten zylinderförmigen Abschnitt mit einem Durchmesser auf, der größer als der Innendurchmesser des Kugellagers ist. Am oberen Ende des ersten Abschnittes ist eine Stufe 27 vorgesehen, so daß eine Fläche 28 gebildet ist, die parallel zur Längsachse der Welle 24 ist. An der Fläche 28 wird ein Spiegel 30 befestigt.
An unteren Ende des ersten Abschnittes schließt koaxial ein zweiter Abschnitt an, der einen Außendurchmesser aufweist, der so gewählt ist, daß der zweite Abschnitt gerade in das Kugellager 23 paßt. Die Länge des zweiten Abschnittes entspricht der Ausdehnung des Kugellagers 23 in axialer Richtung. An den zweiten Abschnitt schließt koaxial ein dritter Abschnitt mit einem kleineren Außendurchmesser als der zweite Abschnitt an. Der dritte Abschnitt weist ein Außengewinde auf. Das zweite Teil 51 weist einen vierten Abschnitt mit einem Durchmesser auf, der größer als der Innendurchmesser des Kugellagers 23 ist und der kleiner als der zweite Durchmesser der zweiten Bohrung 22 ist. An einem Ende des vierten Abschnittes ist eine koaxiale Zylinderbohrung mit einem zu dem Außengewinde des dritten Abschnittes des ersten Teiles 50 passenden Innengewinde vorgesehen. An den vierten Abschnitt des zweiten Teiles schließt koaxial ein fünfter Abschnitt mit einem kleineren Durchmesser als der vierte Abschnitt an. Der fünfte Abschnitt weist in axialer Richtung einen Schlitz auf. An seinem an den vierten Abschnitt angrenzenden Ende ist eine Durchgangsbohrung senkrecht zu der axialen Richtung des fünften Abschnittes angeordnet.
Das erste Teil 50 ist mit seinem zweiten Abschnitt in dem Kugellager 23 angeordnet. Das zweite Teil 51 ist so auf das erste Teil geschraubt, daß die beiden Teile 50, 51 mit dem Innenring des Kugellagers 23 verbunden sind.
In dem ersten Teil 50 der Welle 24 ist in einem Abstand von der der ersten Bohrung 21 zugeordneten Seite der Platte 20 in Richtung zu dem ersten Ende des ersten Teiles 50 der Welle 24 eine dritte Bohrung 25 senkrecht zur Längsachse der Welle 24 vorgesehen. Vorzugsweise ist die dritte Bohrung 25 durchgehend ausgebildet. Ein gerader Federdraht 26 ist parallel zur Plattenebene angeordnet und mit einem Ende in der dritten Bohrung 25 befestigt. Vorzugsweise ist der Federdraht 26 in der dritten Bohrung verklebt.
Ein im wesentlichen zylinderförmiger Stempel 29 weist an einem Ende ein koaxiale Bohrung auf, die einen Durchmesser aufweist, der etwas kleiner als der Durchmesser des fünften Abschnittes des zweiten Teiles 51 ist. Der Stempel ist auf dem fünften Abschnitt aufgesteckt und wird durch die Federkraft des fünften Abschnittes an der Welle 24 fixiert.

Auf der dem ersten Teil 50 der Welle 24 zugeordneten Seite der Platte 20 ist eine Führungsplatte 31 vorgesehen (Fig. 6). In der Führungsplatte 31 sind zwei in einem Abstand voneinander angeordnete Langlöcher 33 vorgesehen. Die Längsachsen der Langlöcher 33 sind parallel zueinander. Innerhalb der Langlöcher 33 ist jeweils ein Führungsstift 34, der jeweils in der Platte 20 befestigt ist, senkrecht zur Plattenebene angeordnet, so daß die Führungsplatte 31 nur in Längsrichtung der Langlöcher 33 auf der Platte 20 gleiten kann. Senkrecht zur Längsrichtung der Langlöcher 33 ist in der Führungsplatte 31 ein Schlitz 32 angeordnet, dessen Ausdehnung in Richtung der Längsachse der Langlöcher 33 größer ist als der Durchmesser des Federdrahtes 26. Auf beiden Seiten des Schlitzes 32 in Richtung der Längsachsen der Langlöcher 33 sind zwei Führungswalzen 35, deren Längsachsen parallel zur Längsachse der Welle 24 sind, so in Richtung der Längsachsen der Langlöcher 33 gegenüberliegend angeordnet, daß ihr minimaler Abstand größer als der Durchmesser des Federdrahtes 26 und kleiner als die Ausdehnung des Schlitzes 32 in Längsrichtung der Langlöcher 33 ist. Die Führungsplatte 31 mit dem Scnlitz 32 ist so auf der Platte 20 vorgesehen, daß ein von der Welle 24 abgewandtes Ende des Federdrahtes 26 zwischen den Führungswalzen 35 angeordnet ist.

Vorzugsweise ist das Spiel zwischen dem Federdraht 26 und den Führungswalzen etwa 0,3mm und der Durchmesser des Federdrahtes 26 zwischen 0,1mm und 0,2 mm.

Auf der Führungsplatte 31 ist eine U-förmige Platte 36 so befestigt, daß die Plattenebenen zueinander parallel sind und beide Schenkel der U-förmigen Platte 36 senkrecht zur Längsrichtung der Langlöcher 33 angeordnet sind und über die Führungsplatte hinausragen. Zwischen den beiden Schenkeln ist eine exzentrische Welle 27 angeordnet, deren Längsachse parallel zur Längsachse der Welle 24 ist. Die Welle 37 ist mit einer Antriebsachse eines Motors 38 exzentrisch verbunden.

Ein quaderförmige Küvettenhalterung 39 weist eine erste Küvettenbohrung 43 und eine daran koaxial anschließende zweite Küvettenbohrung 44 auf, deren Längsachsen senkrecht zu einer ersten Seitenfläche des Quaders sind. Die Küvettenbohrungen 43, 44 erstrecken sich über die gesamte Länge des Quaders in Längsrichtung der Küvettenbohrung 43, 44. Innerhalb der ersten Küvettenbohrung 43 ist eine Küvette 40 vorgesehen. Der Durchmesser der ersten Küvettenbohrung 43 entspricht etwa dem Außendurchmesser der Küvette 40. Der Durchmesser der zweiten Küvettenbohrung ist kleiner als der Durchmesser der ersten Küvettenbohrung. Parallel zu den Küvettenbohrungen 43, 44 sind zwei in einem Abstand voneinander durchgehende vierte Bohrungen 41 angeordnet.

Auf der dem zweiten Teil 51 der Welle 24 zugeordneten Seite der Platte 20 sind zwei in einem Abstand, der dem Abstand der vierten Bohrungen 41 entspricht, voneinander angeordnete Führungsstangen 42 vorgesehen, deren Längsachsen parallel zur Längsachse der Welle 24 sind. Die Führungsstangen 42 sind jeweils einer vierten Bohrung 41 zugeordnet. Der Durchmesser der vierten Bohrungen 41 ist so gewählt, daß die Küvettenhalterung 39 auf den Führungsstangen 42 gleiten kann. Die vierten Bohrungen 41 und die erste Küvettenbohrung 43 sowie die Führungsstangen 42 und die Welle 24 mit dem Stempel 29 sind so angeordnet, daß die Küvette 40 den Stempel 29 zumindest teilweise aufnimmt, wenn die Küvettenhalterung 39 auf die Führungsstangen 42 aufgeschoben ist mit ihrer ersten Seitenfläche mit der dem Stempel 29 zugewandten Seite der Platte 20 in Kontakt ist. In der Küvettenhalterung 39 sind zwei Metallzylinder in einem Abstand voneinander vorgesehen. An den entsprechenden Stellen auf der dem ersten Teil 50 der Welle 24 zugewandten Seite der Platte 20 sind jeweils Magnete 46 vorgesehen. Durch die Magnete 46 und die Metallzylinder 45 ist die Küvettenhalterung 39 magnetisch an der Platte 20 befestigt.

Die Platte 20 und die Küvettenhalterung 39 sind bevorzugt aus Aluminium. Der erste Teil 50 der Welle ist bevorzugt aus Kunststoff. Der zweite Teil 51 der Welle ist bevorzugt aus V2a-Stahl. Die Platte 20 wird bevorzugt elektrisch beheizt und auf einer konstanten Temperatur von etwa 37 °C gehalten. Damit wird die in der Küvette vorgesehene Testflüssigkeit, vorzugsweise Blut, indirekt über die Küvettenhalterung 39 beheizt.

Im Betrieb ist die Küvettenhalterung 39 mit der mit dem Blut gefüllten Küvette 40 an der Platte 20 magnetisch befestigt. Der Stempel 29 ist in Kontakt mit dem Blut. Die exzentrische Welle 37 wird durch den Motor 38 gedreht. Diese Drehbewegung der exzentrischen Welle 37 wird über die U-förmige Platte 36 in eine periodische Bewegung der Führungsplatte 31 in Längsrichtung der Langlöcher 33 umgesetzt. Diese periodische Bewegung der Führungsplatte 31 wird nun wiederum über die Führungswalzen 35 und den Federdraht 26 in eine periodische Drehbewegung der Welle 24 umgesetzt. Wirkt keine Kraft gegen die Drehrichtung der Welle 24 auf den Stempel 29, so führt die Welle 24 die volle periodische Drehbewegung von 4,75° aus. Die Messung des Drehwinkels der Welle 24 erfolgt über den Spiegel 30 nach einer der in dem ersten Ausführungsbeispiel beschriebenen Arten.

Da der Abstand der Führungswalzen 35 in Längsrichtung der Langlöcher 33 größer als der Durchmesser des Federdrahtes 26 ist, besteht zwischen einer Führungswalze 35 und dem Federdraht 26 ein idealerweise punktförmiger Kontakt und es werden dadurch keine Zugkräfte auf den Federdraht 26 ausgeübt. Wenn sich zwischen dem Stempel 29 und der Küvette 40 eine Verbindung, z.B. mittels Netzwerken von Fibrinfäden und Blutplättchen, bildet, wirkt ein Drehmoment gegen die Drehbewegung des Stempels 29. Dieses Drehmoment bewirkt eine Biegung des Federdrahtes 26, wodurch die Welle 24 nicht mehr vollständig ausgelenkt wird. Vielmehr wird ein momentaner Drehwinkel der Welle 24 und damit auch ihre Amplitude bezüglich der periodischen Drehbewegung durch das zu jedem Zeitpunkt gegeben Gleichgewicht zwischen dem durch das Gerinnsel und dem durch die Biegung des Federdrahtes bewirkten Drehmoment bestimmt. Die Amplitude der Drehbewegung der Welle 24 mit dem Stempel 29 stellt also ein Maß für die Stärke des sich bildenden Netzwerkes bzw. Gerinnsels dar.

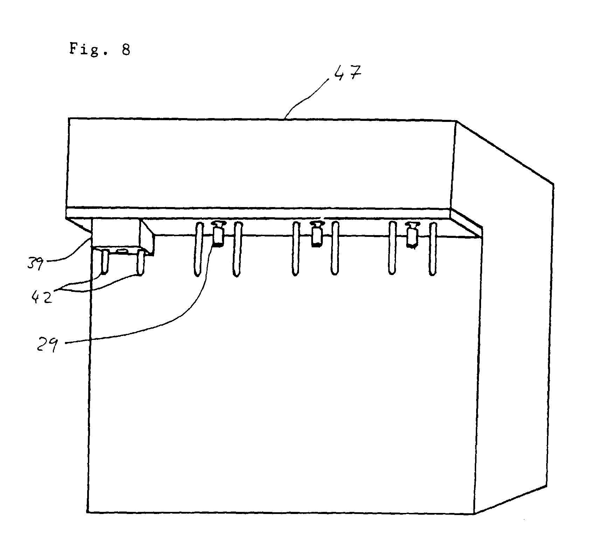

In Fig. 8 ist der Aufbau einer Meßstation gezeigt. Es sind vier in einem Abstand voneinander angeordneten Wellen 24 vorgesehen. In der Führungsplatte sind für jeden Federdraht 26 ein Schlitz 32 mit jeweils zwei Führungswalzen 35 vorgesehen. Der komplette Antrieb der Führungsplatte 31 und die komplette Meßoptik zur Messung der Auslenkung der jeweiligen Welle 24 ist in einem verschlossenen Gehäuse 47 angeordnet. Zur Messung wird nur die Küvette 40 mit der Testflüssigkeit bzw. die Küvettenhalterung ausgewechselt.

In dieser Ausführungsform kann, da die Welle 24 angetrieben und gleichzeitig ihre Auslenkung gemessen wird, das Kugellager 23 im Leerlauf gemessen werden. Damit können besser Fehlmessungen, die durch ein defektes Kugellager bedingt sind, ausgeschlossen werden.

Weiterhin kann in dieser Ausführungsform das komplette optische System, wie in Fig. 8 gezeigt, abgeschlossen gestaltet werden. Zur Messung wird jeweils nur die Küvettenhalterung ausgewechselt. Dadurch ist das gesamte System weniger störanfällig.

Da in dieser Ausführungsform das Kugellager ständig gedreht wird, ist eine genauere Messung möglich, da im Gegensatz zum ersten Ausführungsbeispiel kein Haftmomemt des Kugellagers überwunden werden muß.

Soweit in der Beschreibung Blut als zu messende Flüssigkeit erwähnt wird, kann auch jede andere Testflüssigkeit mit den beschriebenen Ausführungsformen gemessen werden.

## Patentansprüche

1. Vorrichtung zum Messen der Koagulationseigenschaften von Testflüssigkeiten, insbesondere von Blutproben, mit einer Platte (2, 20)
mit einem in der Platte (2, 20), mit seiner Symmetrieachse im wesentlichen senkrecht zur Plattenebene angeordneten Lager (3, 23),
mit einer mit seiner Längsachse im wesentlichen senkrecht zur Plattenebene mit dem Lager (3, 23) verbundenen Welle (1, 24), deren erstes Ende auf einer Seite der Platte (2, 20) und deren zweites Ende auf der gegenüberliegenden Seite der Platte (2, 20) angeordnet ist,
mit einer Einrichtung zum Abtasten der Drehbewegung der Welle (1, 24),
mit einem an dem zweiten Ende der Welle (1, 24) vorgesehenen Stempel (29),
mit einer Küvette (40) zur Aufnahme der Flüssigkeit, deren Innenkontur größer ist als die Außenkontur des Stempels (29), und die zumindest einen Teil des Stempels aufnimmt,
mit einer Vorrichtung zum Drehen von Küvette (40) und Stempel (29) relativ zueinander und
mit einer an der Welle (1, 24) angeordneten Federeinrichtung (4, 26).

2. Vorrichtung nach Anspruch 1, dadurch gekennzeichnet, daß die Vorrichtung zum Drehen die Welle (24) antreibt.

3. Vorrichtung nach Anspruch 1 oder 2, dadurch gekennzeichnet, daß
die Küvette (40) in einer Küvettenhalterung (39) mit einer ersten ebenen Seitenfläche angeordnet ist,
und daß in der Küvettenhalterung (39) zwei im wesentlichen senkrecht zu der ersten Seitenfläche in einem Abstand voneinander angeordnete Durchgangsbohrungen (41) mit einem ersten Durchmesser angeordnet sind,
und daß zwei in dem Abstand voneinander im wesentlichen senkrecht zur Plattenebene vorgesehene Führungsstangen (42) vorgesehen sind, deren eines Ende jeweils mit der Platte verbunden ist und deren anderes Ende sich parallel zu der Welle (24) erstreckt, und die einen Durchmesser aufweisen, der so gewählt ist, daß die Führungsstangen (42) gerade in die Durchgangsbohrungen passen und die Küvettenhalterung (39) auf den Führungsstangen (42) gleiten kann.

4. Vorrichtung nach Anspruch 3, dadurch gekennzeichnet, daß in der Küvettenhalterung nahe der ersten Seitenfläche ein erstes Element vorgesehen ist,
und daß an oder in der Platte (20) ein zweites Element vorgesehen ist, und daß beide Elemente aufeinander eine attraktive Kraft ausüben.

5. Vorrichtung nach Anspruch 4, dadurch gekennzeichnet, daß eines der Elemente ein Magnet ist.

6. Vorrichtung nach einem der Ansprüche 1 bis 5, dadurch gekennzeichnet, daß
eine erstes Ende der Federeinrichtung (4, 26) mit der Welle verbunden ist und daß sich die Federeinrichtung (4, 26) im wesentlichen parallel zur Platte (20) erstreckt.

7. Vorrichtung nach Anspruch 6, dadurch gekennzeichnet, daß ein zweites Ende der Federeinrichtung (26) zwischen zwei im wesentlichen senkrecht zur Plattenebene vorgesehenen Stiften (35) angeordnet ist, und daß zwischen dem zweiten Ende der Federeinrichtung (26) und den zwei Stiften (35) ein Spiel vorgesehen ist.

8. Vorrichtung nach Anspruch 7, dadurch gekennzeichnet, daß auf der Platte (20) gleitend eine Führungsplatte (31) mit zwei in einem Abstand voneinander angeordneten Langlöchern (33), deren Längsachsen parallel sind, vorgesehen ist, und daß in den Langlöchern (33) jeweils ein Führungsstift (34), der mit der Platte (20) verbunden ist, so angeordnet ist, daß die Führungsplatte (31) in Längsrichtung der Langlöcher (33) über einen vorbestimmten Bereich verschiebbar ist.

9. Vorrichtung nach Anspruch 8, dadurch gekennzeichnet, daß die Stifte (35) mittels der Führungsplatte (31) bewegbar sind und
daß an der Führungsplatte (31) ein Aufnehmer mit zwei in einem Abstand voneinander parallelen Schenkeln vorgesehen ist und daß zwischen den Schenkeln ein Exzenter angeordnet ist.

10. Vorrichtung nach Anspruch 1, dadurch gekennzeichnet, daß die Vorrichtung zum Drehen die Küvette (40) antreibt.

11. Vorrichtung nach einem der Ansprüche 1 bis 10, dadurch gekennzeichnet, daß
die Federeinrichtung (4, 26) eine Blattfeder oder ein Federdraht ist.

12. Vorrichtung nach Anspruch 10, dadurch gekennzeichnet, daß die Federeinrichtung (4) eine Spiralfeder ist.

13. Vorrichtung nach Anspruch 12, dadurch gekennzeichnet, daß ein Ende der Spiralfeder (4) fest mit der Platte (2) und das andere Ende fest mit der Welle (1) verbunden ist.

14. Vorrichtung nach einem der Ansprüche 1 bis 13, dadurch gekennzeichnet, daß
das Lager (3, 23) ein Kugellager ist.

15. Vorrichtung nach einem der Ansprüche 1 bis 14, dadurch gekennzeichnet, daß
zumindest ein Teil der Welle (1, 24) aus Aluminium besteht.

16. Vorrichtung nach wenigstens einem der Ansprüche 1 bis 15, dadurch gekennzeichnet, daß
die Welle (1, 24) an ihrem oberen Ende einen Schlitz zur Aufnahme eines Spiegels (5, 30) oder dergleichen aufweist.

17. Vorrichtung nach wenigstens einem der Ansprüche 1 bis 16, dadurch gekennzeichnet, daß
der Spiegel (5, 30) ein Plan- oder Konkavspiegel ist.

## Revendications

1. Dispositif de mesure des propriétés de coagulation de liquides utilisés pour des tests, en particulier d'échantillons de sang, avec une plaque (2, 20), avec un palier (3, 23) disposé dans la plaque (2, 20) ayant un axe de symétrie sensiblement perpendiculaire au plan de la plaque, avec un arbre (1, 24) relié au palier (3, 23) et ayant un axe longitudinal sensiblement perpendiculaire au plan de la plaque, dont la première extrémité est disposée sur une face de plaque (2, 20) et la deuxième extrémité sur la face opposée de plaque (2, 20), avec un dispositif de détection du mouvement de rotation de l'arbre (1, 24), avec un poinçon (29) prévu à la deuxième extrémité de l'arbre (1, 24), avec une cuvette (40) destinée à recevoir le liquide, dont le contour intérieur est plus grand que le contour extérieur du poinçon (29) et qui reçoit au moins une partie du poinçon, avec un dispositif destiné à produire la rotation de la cuvette (40) et du poinçon (29) l'un par rapport à l'autre et avec un dispositif de ressort (4, 26) disposé sur l'arbre (1, 24).

2. Dispositif selon la revendication 1, caractérisé en ce que le dispositif destiné à produire la rotation entraîne l'arbre (24).

3. Dispositif selon la revendication 1 ou 2, caractérisé en ce que la cuvette (40) est disposée dans un logement de cuvette (39) possédant une première surface latérale plane, et en ce qu'il est prévu dans le logement de cuvette (39) deux trous de passage (41) d'un premier diamètre sensiblement perpendiculaires à la première surface latérale et disposés à distance l'un de l'autre, et en ce qu'il est prévu deux tiges de guidage (42) disposées à distance l'une de l'autre et sensiblement perpendiculaires au plan de la plaque, dont chacune a une extrémité reliée à la plaque et l'autre extrémité orientée parallèlement à l'arbre (24), et qui présentent un diamètre choisi de telle sorte que les tiges de guidage (42) entrent juste dans les trous de passage et que le logement de cuvette (39) puisse coulisser sur les tiges de guidage (42).

4. Dispositif selon la revendication 3, caractérisé en ce qu'il est prévu un premier élément dans le logement de cuvette à proximité de la première surface latérale, et en ce qu'il est prévu sur ou dans la plaque (20) un deuxième élément, et en ce que les deux éléments exercent l'un sur l'autre une force d'attraction.

5. Dispositif selon la revendication 4, caractérisé en ce que l'un des éléments est un aimant.

6. Dispositif selon l'une quelconque des revendications 1 à 5, caractérisé en ce qu'une première extrémité du dispositif de ressort (4, 26) est reliée avec l'arbre et le dispositif de ressort (4, 26) est sensiblement parallèle à la plaque (20).

7. Dispositif selon la revendication 6, caractérisé en ce qu'une deuxième extrémité du dispositif de ressort (26) est disposée entre deux goupilles (35) prévues en position sensiblement perpendiculaire au plan de la plaque, et en ce qu'un jeu est prévu entre la deuxième extrémité du dispositif de ressort (26) et les deux goupilles (35).

8. Dispositif selon la revendication 7, caractérisé en ce qu'il est prévu sur la plaque (20) avec possibilité de coulissement une plaque de guidage (31) dotée de deux trous allongés (33) disposés à distance l'un de l'autre et dont les axes longitudinaux sont parallèles, et en ce qu'une goupille de guidage (34) reliée à la plaque (20) est disposée dans chaque trou allongé (33) de telle sorte que la plaque de guidage (31) puisse être déplacée dans le sens longitudinal des trous allongés (33) sur une plage prédéterminée.

9. Dispositif selon la revendication 8, caractérisé en ce que les goupilles (35) sont mobiles au moyen de la plaque de guidage (31) et en ce qu'il est prévu sur la plaque de guidage (31) un dispositif préhenseur doté de deux bras parallèles écartés l'un de l'autre et en ce qu'un excentrique est disposé entre les bras.

10. Dispositif selon la revendication 1, caractérisé en ce que le dispositif produisant la rotation entraîne la cuvette (40).

11. Dispositif selon l'une quelconque des revendications 1 à 10, caractérisé en ce que le dispositif de ressort (4, 26) est un ressort à lame ou un fil d'acier à ressort.

12. Dispositif selon la revendication 10, caractérisé en ce que le dispositif de ressort (4) est un ressort à spirale.

13. Dispositif selon la revendication 12, caractérisé en ce qu'une extrémité du ressort à spirale (4) est fixée à la plaque (2) et l'autre extrémité fixée à l'arbre (1).

14. Dispositif selon l'une quelconque des revendications 1 à 13, caractérisé en ce que le palier (3, 23) est un roulement à billes.

15. Dispositif selon l'une quelconque des revendications 1 à 14, caractérisé en ce qu'une partie au moins de l'arbre est composée d'aluminium.

16. Dispositif selon l'une ou l'ensemble des revendications 1 à 15, caractérisé en ce que l'arbre (1, 2, 4) présente à son extrémité supérieure une fente destinée à recevoir un miroir (5, 30) ou similaire.

17. Dispositif selon l'une ou l'ensemble des revendications 1 à 16, caractérisé en ce que le miroir (5, 30) est un miroir plan ou concave.

## Claims

1. Apparatus for measuring the coagulation properties of test fluids, in particular of blood samples, having a plate (2, 20) having a bearing (3, 23) arranged in the plate (2, 20), with its axis of symmetry substantially perpendicular to the plane of the plate,
having a shaft (1, 24) connected to the bearing (3, 23), with its longitudinal axis substantially perpendicular to the plane of the plate, the first end of which shaft is arranged on one side of the plate (2, 20) and the second of which is arranged on the opposite side of the plate (2, 20),
having a means for sensing the rotary movement of the shaft (1, 24),
having a plunger (29) provided at the second end of the shaft (1, 24),
having a cuvette (40) for receiving the fluid, the inner contour of which is greater than the outer contour of the plunger (29), and which receives at least a part of the plunger,
having a device for rotation of cuvette (40) and plunger (29) relative to each other and
having a spring means (4, 26) arranged on the shaft (1, 24).

2. Apparatus according to Claim 1, characterised in that the device for rotation drives the shaft (24).

3. Apparatus according to Claim 1 or 2, characterised in that the cuvette (40) is arranged in a cuvette holder (39) having a first plane side face, and in that two through bores (41) having a first diameter, which are arranged substantially perpendicularly to the first side face at a distance from each other, are provided in the cuvette holder (39), and in that two guide rods (42), one end of which is in each case connected to the plate and the other end of which extends parallel to the shaft (24), and which have a diameter chosen so that the guide rods (42) just fit into the through bores and the cuvette holder (39) is able to slide on the guide rods (42), are provided at the distance from each other substantially perpendicularly to the plane of the plate.

4. Apparatus according to Claim 3, characterised in that a first element is provided in the cuvette holder close to the first side face, and in that a second element is provided on or in the plate (20), and in that the two elements exert an attractive force on each other.

5. Apparatus according to Claim 4, characterised in that one of the elements is a magnet.

6. Apparatus according to one of Claims 1 to 5, characterised in that a first end of the spring means (4, 26) is connected to the shaft and in that the spring means (4, 26) extends substantially parallel to the plate (20).

7. Apparatus according to Claim 6, characterised in that a second end of the spring means (26) is arranged between two pins (35) provided substantially perpendicularly to the plane of the plate, and in that a clearance is provided between the second end of the spring means (26) and the two pins (35).

8. Apparatus according to Claim 7, characterised in that a guide plate (31), having two elongated holes (33) which are arranged at a distance from each other and the longitudinal axes of which are parallel, is provided slidingly on the plate (20), and in that a guide pin (34), which is connected to the plate (20), is arranged in each of the elongated holes (33) so that the guide plate (31) is displaceable in the longitudinal direction of the elongated holes (33) over a predetermined range.

9. Apparatus according to Claim 8, characterised in that the pins (35) are movable by means of the guide plate (31) and in that a receiver having two parallel legs at a distance from each other is provided on the guide plate (31) and in that an eccentric is arranged between the legs.

10. Apparatus according to Claim 1, characterised in that the device for rotation drives the cuvette (40).

11. Apparatus according to one of Claims 1 to 10, characterised in that the spring means (4, 26) is a leaf spring or a spring wire.

12. Apparatus according to Claim 10, characterised in that the spring means (4) is a spiral spring.

13. Apparatus according to Claim 12, characterised in that one end of the spiral spring (4) is fixedly connected to the plate (2) and the other end is fixedly connected to the shaft (1).

14. Apparatus according to one of Claims 1 to 13, characterised in that the bearing (3, 23) is a ball bearing.

15. Apparatus according to one of Claims 1 to 14, characterised in that at least part of the shaft (1, 24) consists of aluminium.

16. Apparatus according to at least one of Claims 1 to 15, characterised in that the shaft (1, 24) has at its upper end a slot for receiving a mirror (5, 30) or the like.

17. Apparatus according to at least one of Claims 1 to 16, characterised in that the mirror (5, 30) is a plane or concave mirror.
